# FASCICULE DE BREVET EUROPEEN

(11) **EP 1 899 269 B1**
(45) Date de publication et mention de la délivrance du brevet: **21.11.2018**
(21) Numéro de dépôt: 06764819.6
(22) Date de dépôt: 22.06.2006
(51) Int. Cl.: B01J 29/06, B01J 29/18, B01J 29/65, B01J 29/70, B01J 37/00, C07C 2/12, C10G 50/00, C01B 39/02, C01B 39/06

(54) **PROCEDE D'OLIGOMÉRISATION D'UNE CHARGE OLÉFINIQUE**
VERFAHREN ZUR OLIGOMERISIERUNG EINER OLEFIN CHARGE
PROCESS FOR OLIGOMERIZING AN OLEFINIC FEEDSTOCK

(30) Priorité: 28.06.2005 FR 0506588
(43) Date de publication de la demande: 19.03.2008
(73) Titulaire: IFP Energies nouvelles, 92500 Rueil-Malmaison (FR)
(72) Inventeur: SIMON, Laurent, 69003 Lyon (FR); LACOMBE, Sylvie, F-69230 Saint Genis Laval (FR)
(74) Mandataire: IFP Energies nouvelles
(86) Numéro de dépôt international: PCT/FR2006/001427
(87) Numéro de publication internationale: WO 2007/003737

(56) Documents cités:
- EP-A- 0 493 931
- EP-A- 0 757 976
- WO-A-02/04575
- WO-A-2005/058777
- FR-A- 2 477 903

## Description

### Domaine technique

La présente invention se rapporte à un procédé d'oligomérisation d'une charge oléfinique contenant des molécules hydrocarbonées ayant de 2 à 12 atomes de carbone par molécule réalisé en présence d'un catalyseur contenant une zéolithe traitée laquelle est préparée selon un procédé de traitement. Ledit procédé de traitement d'au moins une zéolithe présentant des petits et / ou des moyens pores, en particulier de taille inférieure ou égale à 7 Å afin d'obtenir une zéolithe modifiée qui est avantageusement utilisée dans un catalyseur et mis en oeuvre dans différents procédés de conversion chimiques des hydrocarbures. L'invention se rapporte à l'utilisation d'un catalyseur contenant la zéolithe modifiée dans un procédé d'oligomérisation d'une charge oléfinique légère.

### Etat de la technique antérieure

Plusieurs brevets ont déjà fait état de méthodes pour modifier des zéolithes. En particulier, le brevet US 4,402,867 décrit une méthode de préparation d'un catalyseur à base de zéolithe comprenant une étape consistant à déposer en phase aqueuse au moins 0,3% poids de silice amorphe à l'intérieur des pores de la zéolithe. Le brevet US 4,996,034 décrit un procédé de substitution d'atomes d'aluminium présents dans une charpente zéolithique par des atomes de silicium, ledit procédé étant réalisé en une étape en milieu aqueux utilisant des sels de fluorosilicates. Le brevet US 4,451,572 décrit la préparation d'un catalyseur zéolithique comprenant une étape de dépôt de matières organosiliciques en phase vapeur ou liquide, les zéolithes visées étant des zéolithes à larges pores, en particulier la zéolithe Y.

Le brevet US 5,057,640 décrit un procédé d'oligomérisation du propylène mettant en oeuvre un catalyseur contenant une zéolithe de rapport Si/Al supérieur à 12 et de "Constraint Index" (indice de contrainte, Cl) compris entre 1 et 12 et dans lequel au moins 0,1% en poids de silice par rapport au poids de la zéolithe a été ajouté. Le catalyseur visé dans ce brevet US 5,057,640 présente une adsorption de n-hexane de 1% de moins que sur la zéolithe de départ.

Le document EP 0 757 976 A1 divulgue un procédé pour l'oligomérisation des oléfines ayant de 2 à 12 atomes de carbone par molécule en présence d'un catalyseur comprenant une zéolithe modifiée formée de cristaux sous forme d'aiguilles. En particulier, l'objectif de EP 0 757 976 est la production d'oléfines en C5-C20 et de préférence en C5-C15 à partir de propylène et/ou de butène dans le but de soumettre ensuite les oléfines produites à une étape d'hydrofomylation pour produire des aldéhydes et des alcools qui pourront ensuite être utilisés pour produire des plastifiants, des esters, des mercaptans et des surfactants.

Un des critères essentiels de l'invention selon EP 0 757 976 est la mise en oeuvre du procédé d'oligomérisation en présence de 0,05 à 0,25% d'eau et en présence d'un catalyseur à base de zéolithe modifiée formée de cristaux sous forme d'aiguille présentant une taille particulière et ayant subie une étape de calcination réalisée dans des conditions telles que le composé organique (structurant organique) utilisé pour la synthèse de ladite zéolithe reste dans les pores des cristaux de la zéolithe c'est-à-dire à une température comprise entre 120 et 430°C (page 4 ligne 15-17, ligne 21 et lignes 44-45). Le catalyseur peut contenir une zéolithe qui a été soumise à un traitement par désalumination (page 3 lignes 40-41). Par ailleurs, la zéolithe modifiée présente dans le catalyseur utilisé dans le procédé selon EP 0 757 976 n'a nullement été soumise à un traitement en présence d'un composé moléculaire à base de silicium.

### Résumé

La présente invention concerne un procédé d'oligomérisation d'une charge oléfinique contenant des molécules hydrocarbonées ayant de 2 à 12 atomes de carbone par molécule réalisé en présence d'un catalyseur contenant une zéolithe traitée laquelle est préparée selon un procédé de traitement. Ledit procédé de traitement d'au moins une zéolithe présentant une taille de pores inférieure ou égale à 7 Å comporte au moins a) une étape de désalumination de ladite zéolithe, b) une étape d'échange cationique par au moins un cation autre que H⁺, c) une étape de traitement de ladite zéolithe obtenue à l'étape b) en présence d'au moins un composé moléculaire contenant au moins un atome de silicium et d) au moins une étape de traitement thermique. Ladite zéolithe est de préférence choisie parmi les zéolithes de type structural MEL, MFI, ITH, NES, EUO, ERI, FER, CHA, MFS, MWW, MTT, TON et MOR. Il est préféré que la zéolithe issue de l'étape b) soit dépourvue de tout proton afin de réaliser l'étape c) de manière optimale. L'étape c) du procédé selon l'invention est de préférence réalisée en procédant au dépôt dudit composé moléculaire contenant au moins un atome de silicium en phase gazeuse et est mise en oeuvre dans un réacteur à lit fixe.

### Intérêt

Il a été découvert, de manière surprenante, qu'un catalyseur comprenant une zéolithe modifiée par un procédé de traitement comportant au moins a) une étape de désalumination de ladite zéolithe, b) une étape d'échange cationique par au moins un cation autre que H⁺, c) une étape de traitement de ladite zéolithe obtenue à l'étape b) en présence d'au moins un composé moléculaire contenant au moins un atome de silicium et d) au moins une étape de traitement thermique conduit à des performances catalytiques améliorées, notamment en termes d'activité et de sélectivité dans une réaction d'oligomérisation d'une charge oléfinique contenant des molécules hydrocarbonées ayant de 2 à 12 atomes de carbone par molécule, de préférence de 3 à 7 atomes de carbone par molécule, et de manière très préférée contenant de 4 à 6 atomes de carbone par molécule. En particulier, un tel catalyseur permet d'augmenter notablement l'activité catalytique conduisant ainsi à une notable augmentation de la conversion de la charge oléfinique et une augmentation des rendements des coupes essence et diesel par rapport à ceux obtenus en employant un catalyseur de l'état de la technique. L'indice de cétane qui traduit la linéarité des chaînes hydrocarbonées présentes dans le diesel est également avantageusement amélioré par rapport à celui que présente généralement une coupe diesel. L'utilisation du catalyseur tel que décrit ci-dessus dans un procédé d'oligomérisation d'une charge oléfinique contenant des molécules hydrocarbonées ayant de 2 à 12 atomes de carbone par molécule, de préférence de 3 à 7 atomes de carbone par molécule, et de manière très préférée contenant de 4 à 6 atomes de carbone par molécule permet la production d'un oligomérat de très bonne qualité qui peut avantageusement être directement intégré au pool gazole d'une raffinerie.

### Description de l'invention

La présente invention a pour objet un procédé d'oligomérisation d'une charge oléfinique contenant des molécules hydrocarbonées ayant de 2 à 12 atomes de carbone par molécule réalisé en présence d'un catalyseur contenant une zéolithe traitée laquelle est préparée selon un procédé de traitement. Ledit procédé de traitement d'au moins une zéolithe présentant une taille de pores inférieure ou égale à 7 Å comporte au moins :
a) une étape de désalumination de ladite zéolithe,
b) une étape d'échange cationique par au moins un cation autre que H⁺,
c) une étape de traitement de ladite zéolithe obtenue à l'étape b) en présence d'au moins un composé moléculaire contenant au moins un atome de silicium,
d) au moins une étape de traitement thermique.

Conformément à l'invention, les zéolithes traitées selon le procédé de l'invention présente une taille de pores inférieure ou égale à 7 Å et de manière préférée inférieure à 6,5 Å. Les zéolithes sont celles définies dans la classification "Atlas of Zeolite Structure Types", W. M Meier, D. H. Oison and Ch. Baerlocher, 5th revised edition, 2001, Elsevier" auquel se réfère également la présente demande mais peuvent également être toute zéolithe présentant une taille de pores inférieure ou égale à 7 Å . Les zéolithes répertoriées dans l' "Atlas of Zeolite Structure Types" y sont classées selon la taille de leurs ouvertures de pores ou de canaux.

Toutes les zéolithes présentant une taille de pores inférieure ou égale à 7 Å et de manière préférée inférieure à 6,5 Å conviennent pour la mise en oeuvre du procédé de traitement. De manière avantageuse, la zéolithe à traiter selon le procédé de l'invention présente soit au moins des canaux dont l'ouverture est définie par un anneau à 8 atomes d'oxygène (8 MR) soit au moins des canaux dont l'ouverture est définie par un anneau à 10 atomes d'oxygène (10 MR) soit encore à la fois des canaux dont l'ouverture est définie par un anneau à 8 atomes d'oxygène (8 MR) et des canaux dont l'ouverture est définie par un anneau à 10 atomes d'oxygène (10 MR), lesdits canaux pouvant être interconnectés. Une zéolithe présentant au moins des canaux dont l'ouverture est définie par un anneau à 12 atomes d'oxygène (12 MR) convient également pour la mise en oeuvre du procédé de traitement dès lors qu'elle présente une taille de pores inférieure ou égale à 7 Å. En particulier, une zéolithe de type structural MOR qui présente à la fois des canaux dont l'ouverture est définie par un anneau à 8 atomes d'oxygène (8 MR) et des canaux dont l'ouverture est définie par un anneau à 12 atomes d'oxygène (12 MR) convient pour la mise en oeuvre du procédé de traitement.
La zéolithe à traiter selon le procédé de l'invention contient au moins du silicium et de l'aluminium dans une proportion telle que le rapport atomique Si/Al est préférentiellement compris entre 2 et 200, de manière plus préférée compris entre 5 et 100 et de manière encore plus préférée compris entre 8 et 80. Elle contient avantageusement au moins un autre élément W, différent du silicium et de l'aluminium, s'intégrant sous forme tétraédrique dans la charpente de la zéolithe. De préférence, ledit élément W est choisi parmi le fer, le germanium, le bore et le titane et représente une portion pondérale comprise entre 5 et 30% de l'ensemble des atomes constitutifs de la charpente zéolithique autre que les atomes d'oxygène. La zéolithe présente alors un rapport (Si+W)/Al compris entre 2 et 200, de préférence compris entre 5 et 100 et de manière très préférée compris entre 8 et 80, W étant défini comme précédemment.

La zéolithe à traiter selon le procédé de l'invention est de préférence choisie parmi les zéolithes de type structural MEL, MFI, ITH, NES, EUO, ERI, FER, CHA, MFS, MWW, MTT, TON et MOR et de manière très préférée ladite zéolithe est choisie parmi les zéolithes de type structural TON, MFS, MOR et FER. Parmi les zéolithes de type structural MEL, la zéolithe ZSM-11 est préférée. Parmi les zéolithes de type structural MFI, la zéolithe ZSM-5 est préférée. Parmi les zéolithes de type structural ITH, la zéolithe ITQ-13 est préférée (US 6.471.941). Parmi les zéolithes de type structural NES, la zéolithe NU-87 est préférée. Parmi les zéolithes de type structural EUO, la zéolithe EU-1 est préférée. Parmi les zéolithes de type structural ERI, la zéolithe erionite est préférée. Parmi les zéolithes de type structural FER, les zéolithes ferriérite et ZSM-35 sont préférées. Parmi les zéolithes de type structural CHA, la zéolithe chabazite est préférée. Parmi les zéolithes de type structural MFS, la zéolithe ZSM-57 est préférée. Parmi les zéolithes de type structural MWW, la zéolithe MCM-22 est préférée. Parmi les zéolithes de type structural MTT, la zéolithe ZSM-23 est préférée. Parmi les zéolithes de type structural TON, la zéolithe ZSM-22 est préférée. Parmi les zéolithes de type structural MOR, la zéolithe mordénite est préférée. Ces zéolithes et leur mode de préparation sont bien connus de l'Homme du métier.

L'étape a) du procédé selon l'invention est une étape de désalumination consistant à extraire partiellement des atomes d'aluminium de la charpente zéolithique. D'une manière préférée, le rapport atomique Si/Al de la zéolithe désaluminée, obtenue à l'issue de ladite étape a), est augmenté d'au moins 10 % par rapport au rapport atomique Si/Al initial de la zéolithe à traiter selon le procédé de l'invention, et de manière très préférée ladite étape a) du procédé selon l'invention conduit à une augmentation d'au moins 20 % du rapport atomique Si/Al initial de la zéolithe à traiter selon le procédé de l'invention.

L'étape a) de désalumination du procédé selon l'invention peut être réalisée par toute méthode connue de l'Homme du métier. De manière avantageuse, elle est réalisée par l'une ou l'autre des deux méthodes décrites ci-après, la première méthode de désalumination étant dite méthode de l'attaque acide et la deuxième méthode de désalumination étant dite méthode de traitement thermique.

Préalablement à la mise en oeuvre de ladite étape a), la zéolithe à traiter selon le procédé de l'invention peut se trouver soit sous sa forme brute de synthèse, contenant encore le structurant organique utilisé pour la préparer soit sous forme calcinée. De manière très préférée, elle se présente sous forme calcinée et contient au moins un cation, de préférence un proton de telle sorte qu'elle se trouve sous sa forme protonée (forme hydrogène H⁺) dans laquelle la teneur en sodium est inférieure à 0,2 % poids, de préférence inférieure à 0,1 % poids et de manière très préférée inférieure à 0,05 % poids par rapport au poids total de zéolithe sèche. Pour obtenir une zéolithe sous sa forme protonée, préalablement à la mise en oeuvre de ladite étape a), on procède généralement à un ou plusieurs échange(s) ionique(s) par une solution contenant au moins un sel d'ammonium, par exemple le nitrate d'ammonium NH₄NO₃, de manière à éliminer au moins en partie, de préférence pratiquement totalement, un cation alcalin présent dans la zéolithe. Une étape de calcination sous flux d'air sec, à une température généralement comprise entre environ 400 et 500°C, a ensuite pour but de régénérer les protons de la zéolithe par désorption d'ammoniaque conduisant ainsi à la forme hydrogène de la zéolithe.

Une première méthode pour réaliser l'étape a) du procédé selon l'invention est la méthode dite de l'attaque acide directe. Elle s'applique préférentiellement lorsque la zéolithe à désaluminer se trouve sous sa forme hydrogène H⁺ et est débarrassée du structurant organique. Ladite méthode comprend une étape de traitement par une solution aqueuse d'un acide minéral tel que HNO₃ ou HCl ou organique tel que CH₃CO₂H. Cette étape peut être répétée autant de fois qu'il est nécessaire afin d'obtenir le niveau de désalumination voulu. Pour atteindre le rapport Si/Al désiré, il est nécessaire de bien choisir les conditions opératoires ; de ce point de vue les paramètres les plus critiques sont la température de traitement par la solution aqueuse d'acide qui est avantageusement comprise entre 25°C et 100°C, la concentration dudit acide qui est avantageusement comprise entre 1N et 15N, la nature dudit acide qui est préférentiellement choisi parmi HNO₃, HCl et CH₃CO₂H, le rapport entre la quantité de solution acide et le volume de zéolithe traitée qui est préférentiellement compris entre 1 et 30, la durée de traitement qui est comprise préférentiellement entre 30 minutes et 10 heures et le nombre de traitement réalisés qui est compris préférentiellement entre 1 et 5.

On peut également effectuer des traitements de désalumination par des composés chimiques désaluminants tels que, à titre d'exemples et de façon non exhaustive, le tétrachlorure de silicium (SiCl₄) (Sohn et al. Applied Catalysis A-General, 218 (1-2), 229-234 (2001)), l'hexafluorosilicate d'ammonium ((NH₄)₂SiF₆) (Garralon et al., Zeolites, 8, 268-272 (1988)), l'acide éthylènediaminetétracétique (EDTA) (Gola et al., Microporous and Mesoporous Materials, 40 (1-3), 73-83 (2000)) ainsi que sa forme mono et disodique. Ces réactifs peuvent être utilisés en solution ou en phase gaz par exemple dans le cas de SiCl₄.

Une deuxième méthode pour réaliser l'étape a) du procédé selon l'invention est la méthode dite de traitement thermique (en particulier à la vapeur d'eau ou "steaming"). Cette méthode comprend au moins un cycle de désalumination de charpente lequel comporte au moins un traitement thermique réalisé à une température généralement comprise entre 550 et 900°C en présence d'un gaz vecteur, généralement l'azote et en présence ou non d'oxygène. Ledit traitement thermique est préférentiellement réalisé en présence de vapeur d'eau et est avantageusement suivi d'au moins une attaque acide par une solution aqueuse d'un acide minéral, tel que HNO₃ ou HCl ou organique tel que CH₃CO₂H. Pendant le traitement thermique, le gaz vecteur pourra contenir préférentiellement entre 10 et 90% et très préférentiellement entre 20 et 80% de vapeur d'eau. La durée du traitement thermique, préférentiellement réalisé en présence de vapeur d'eau, est avantageusement comprise entre 1 et 10 heures et très avantageusement comprise entre 1 heure et 7 heures. Les conditions de l'attaque acide qui suit avantageusement le traitement thermique sont adaptées de manière à obtenir le niveau de désalumination souhaité. La température de traitement par la solution aqueuse d'acide est préférentiellement comprise entre 25°C et 100°C, la concentration dudit acide est préférentiellement comprise entre 1N et 15N, ledit acide étant avantageusement choisi parmi HNO₃, HCl, ou CH₃CO₂H, le rapport entre la quantité de solution acide et le volume de zéolithe traitée est préférentiellement compris entre 1 et 30, la durée de traitement de l'attaque acide est préférentiellement comprise entre 30 minutes et 10 heures et le nombre de traitements à l'attaque acide réalisés est préférentiellement compris entre 1 et 5. Dans le même but on peut aussi jouer sur le nombre de cycles de désalumination traitement thermique - attaque acide qui sont effectués.

Cette deuxième méthode pour réaliser l'étape a) du procédé selon l'invention s'applique aussi bien pour désaluminer une zéolithe brute de synthèse, contenant encore le structurant organique, que pour désaluminer une zéolithe sous sa forme calcinée et débarrassée du structurant organique. En particulier, lorsqu'il s'agit de réaliser ladite étape a) sur une zéolithe contenant encore le structurant organique, le traitement thermique, avantageusement réalisé à une température généralement comprise entre 550°C et 900°C et préférentiellement mis en oeuvre en présence de vapeur d'eau permet la calcination simultanée du structurant organique.

Une variante de cette deuxième méthode consiste à remplacer l'étape, éventuelle, d'attaque acide, c'est-à-dire du traitement par une solution d'acide, par un traitement par une solution d'un composé chimique désaluminant tels que, par exemple, le tétrachlorure de silicium (SiCl₄) (Sohn et al. Applied Catalysis A-General, 218 (1-2), 229-234 (2001)), l'hexafluorosilicate d'ammonium ((NH4)₂SiF₆), (Garralon et al., Zeolites, 8, 268-272 (1988)), l'acide éthylènediaminetétracétique (EDTA) (Gola et al., Microporous and Mesoporous Materials, 40 (1-3), 73-83 (2000)) ainsi que sa forme mono et disodique. Ces réactifs peuvent être utilisés en solution ou en phase gaz par exemple dans le cas de SiCl₄.
Le cycle de désalumination de la charpente, comportant au moins une étape de traitement thermique préférentiellement réalisé en présence de vapeur d'eau et préférentiellement suivi d'au moins une étape d'attaque en milieu acide de la zéolithe, peut être répété autant de fois qu'il est nécessaire pour obtenir la zéolithe désaluminée possédant les caractéristiques désirées, notamment en terme de rapport atomique Si/Al. De même, suite au traitement thermique préférentiellement réalisé en présence de vapeur d'eau, plusieurs attaques acides successives, avec des solutions en acide de concentrations éventuellement différentes, peuvent être opérées.

A l'issue de l'étape a) de désalumination du procédé selon l'invention, la zéolithe désaluminée se présente sous forme cationique, de préférence sous forme protonée (forme hydrogène H⁺) et est débarrassée du structurant organique. Le cation présent dans la zéolithe désaluminée à l'issue de l'étape a) est un cation dit cation de compensation comme cela est bien connu de l'Homme du métier.
La zéolithe désaluminée sous forme cationique, de préférence sous forme protonée, est ensuite soumise, conformément à l'étape b) du procédé selon l'invention, à une étape d'échange cationique par au moins un cation autre que H⁺. De préférence, il s'agit d'un cation non acide. L'échange cationique selon l'étape b) du procédé selon l'invention peut être réalisé par toutes les techniques connues de l'Homme du métier, et en particulier par échange en solution en excès. Le cation employé pour réaliser cet échange cationique avec le cation de compensation peut être de tout type et de tout volume afin de permettre l'obtention de différentes caractéristiques texturales de la zéolithe finale. Le cation préféré pour réaliser ladite étape b) est choisi parmi les métaux des groupes IA et IIA de la classification périodique des éléments, et plus particulièrement ledit cation est choisi parmi les cations Na⁺, Li⁺, K⁺, Rb⁺, Cs⁺, Ba²⁺ et Ca²⁺. Il est également avantageux pour la mise en oeuvre du procédé selon l'invention d'échanger le cation de compensation par un ion ammonium NH₄⁺. La zéolithe obtenue à l'issue de l'étape b) du procédé selon l'invention est une zéolithe échangée. Le taux d'échange par rapport au proton est supérieur à 95 %.

La zéolithe échangée selon l'étape b) et préférentiellement dépourvue de tout cation H⁺ est prête pour subir une sélectivation de l'acidité de ses surfaces interne et externe. Par "sélectivation", on entend au sens de la présente invention, la neutralisation de l'acidité de la surface externe et de la surface interne de chacun des cristaux de la zéolithe. La neutralisation de l'acidité peut se faire par toute méthode connue de l'Homme du métier. Les méthodes conventionnelles emploient généralement pour réaliser la sélectivation de zéolithes des molécules dont le diamètre cinétique est inférieur au diamètre de l'ouverture des pores de la zéolithe désaluminée.

Les molécules généralement utilisées pour passiver ou sélectiver les surfaces externe et interne de la zéolithe sont des composés contenant des atomes pouvant interagir avec les sites de surfaces externe et interne de chacun des cristaux de la zéolithe. Les molécules utilisées sont des molécules organiques ou inorganiques contenant un ou plusieurs atome(s) de silicium. Aussi, conformément à l'étape c) du procédé de traitement, la zéolithe échangée selon l'étape b) du procédé de l'invention est soumise à une étape de traitement en présence d'au moins un composé moléculaire contenant au moins un atome de silicium. Ladite étape c) permet le dépôt d'une couche de silice amorphe sur les surfaces externe et interne de chacun des cristaux de la zéolithe conduisant à une diminution au moins partielle, de préférence complète, de l'acidité de Lewis. L'acidité de Lewis est caractérisée par infrarouge en adsorbant du CO. L'intégration de la bande entre 2230 et 2143 cm⁻¹ permet de mettre en évidence la variation d'acidité de Lewis sur la zéolithe avant et après le traitement selon l'étape c) du procédé selon l'invention. La perte d'acidité de Lewis est supérieure à 30%. Le composé moléculaire utilisé dans ladite étape c) comprend de préférence au plus 2 atomes de silicium par molécule. De manière préférée, le composé moléculaire contenant au moins un atome de silicium est choisi parmi les composés de formule Si-R₄ et Si₂-R₆ où R peut être soit de l'hydrogène, soit un groupe alkyle, aryle ou acyle, soit un groupe alkoxy (O-R'), soit un groupe hydroxyl (-OH) soit encore un halogène. Au sein d'une même molécule Si-R₄ ou Si₂-R₆, le groupement R peut être soit identique soit différent. Par exemple, on pourra d'après les formules décrites ci-dessus choisir des composés moléculaires de formule SiH₄, Si₂H₆ ou Si(C₂H₅)₃(CH₃). Ainsi, le composé moléculaire contenant au moins un atome de silicium employé dans l'étape c) du procédé selon l'invention peut être un composé de type silane, disilane, alkylsilane, alkoxysilane ou siloxane. Ledit composé moléculaire présente un diamètre cinétique inférieur au diamètre de l'ouverture des pores de la zéolithe désaluminée.

Ladite étape c) du procédé selon l'invention qui consiste à traiter la zéolithe échangée selon l'étape b) en présence d'au moins un composé moléculaire contenant au moins un atome de silicium est réalisée par dépôt dudit composé sur les surfaces interne et externe de la zéolithe. On peut procéder à un dépôt en phase gazeuse appelé dépôt CVD ("Chemical Vapor Déposition") ou à un dépôt en phase liquide appelé dépôt CLD ("Chemical Liquid Déposition") par toutes les méthodes connues de l'homme du métier. De manière préférée, ladite étape c) est réalisée en procédant au dépôt dudit composé moléculaire contenant au moins un atome de silicium en phase gazeuse.

Un dépôt CLD peut être fait soit en milieu aqueux soit dans un solvant organique. Lors de l'imprégnation en milieu aqueux du composé moléculaire contenant au moins un atome de silicium, on pourra ou non ajouter un ou plusieurs tensioactif(s) dans la solution d'imprégnation. Le dépôt CLD est bien connu de l'Homme du métier (Chon et al., Studies Surface Science and Catalysis, vol. 105, 2059-2065, 1997).

De manière préférée, le dépôt du composé moléculaire contenant au moins un atome de silicium sur les surfaces interne et externe de la zéolithe est réalisé en phase gazeuse. L'étape c) selon le procédé de l'invention est réalisée dans un réacteur à lit fixe. Préalablement à la réaction de dépôt en phase gazeuse (CVD) dans ledit réacteur à lit fixe, la zéolithe est préférentiellement activée. L'activation de la zéolithe dans le réacteur à lit fixe est réalisée sous oxygène, sous air ou sous gaz inerte, ou sous un mélange d'air et de gaz inerte ou d'oxygène et de gaz inerte. La température d'activation de la zéolithe est avantageusement comprise entre 100 et 600°C, et très avantageusement entre 200°C et 550°C. Le composé moléculaire contenant au moins un atome de silicium devant être déposé sur les surfaces interne et externe de chacun des cristaux de la zéolithe est envoyé dans le réacteur en phase vapeur, ledit composé moléculaire étant dilué dans un gaz vecteur qui peut être soit de l'hydrogène (H₂), soit de l'air, soit de l'Argon (Ar), soit de l'hélium (He), soit encore de l'azote (N₂), préférentiellement le gaz vecteur est un gaz inerte choisi parmi Ar, He, et N₂. Pour obtenir une couche de silice amorphe de qualité optimale sur les surfaces interne et externe de la zéolithe, il est nécessaire de bien choisir les conditions opératoires pour le dépôt du composé moléculaire contenant au moins un atome de silicium. En particulier, la température du lit de zéolithe pendant le dépôt est préférentiellement comprise entre 10°C et 300°C, et très préférentiellement comprise entre 25 et 200°C, la pression partielle dans la phase gaz du composé moléculaire à déposer sur les surfaces interne et externe de la zéolithe est préférentiellement comprise entre 0,0001 et 0,5 bar, et très préférentiellement comprise entre 0,001 et 0,2 bar, la durée du dépôt est préférentiellement comprise entre 10 minutes et 10 heures et très préférentiellement comprise entre 30 minutes et 5 heures et encore plus préférentiellement entre 1 heure et 3 heures.

Conformément à l'étape d) du procédé selon l'invention, le composé moléculaire contenant au moins un atome de silicium est décomposé par un traitement thermique lequel est réalisé à une température préférentiellement comprise entre 200 et 700°C, plus préférentiellement entre 300 et 500°C. Ladite étape de traitement thermique est mise en oeuvre sous air, sous oxygène, sous hydrogène, sous azote ou sous argon ou sous un mélange d'azote et d'argon, ladite étape pouvant être éventuellement réalisée en présence de vapeur d'eau. La durée de ce traitement est avantageusement comprise entre 2 heures et 5 heures.

Dans le cas où l'étape b) d'échange cationique du procédé selon l'invention est réalisée avec un cation ammonium NH₄⁺, ladite étape d) consistant en un traitement thermique réalise simultanément la décomposition du composé moléculaire employé pour la mise en oeuvre de ladite étape c) du procédé de l'invention et l'obtention de la zéolithe modifiée par le traitement du procédé selon l'invention, sous sa forme hydrogène.

Dans le cas où l'étape b) d'échange cationique du procédé selon l'invention est réalisée avec un cation différent de H⁺, préférentiellement choisi parmi les métaux des groupes IA et IIA, et différent de NH₄⁺, l'étape d) du procédé selon l'invention décrite ci-dessus est suivie d'une étape e) d'échange dudit cation par un cation ammonium, ladite étape e) étant elle-même suivie d'une étape f) consistant en un traitement thermique.
L'étape e) comprend un ou plusieurs échange(s) ionique(s) réalisé par une solution contenant au moins un sel d'ammonium, par exemple le nitrate d'ammonium NH₄NO₃, de manière à éliminer au moins en partie, de préférence pratiquement totalement voire totalement, le cation de compensation encore présent dans la zéolithe issue de l'étape d) du procédé selon l'invention et introduit au cours de l'étape b) du procédé de l'invention. L'étape e) est suivie d'une étape f) consistant en un traitement thermique réalisé préférentiellement sous flux d'air sec, à une température avantageusement comprise entre environ 400 et 500°C. Ladite étape f) a pour but d'obtenir la zéolithe traitée selon le procédé de l'invention sous sa forme hydrogène H⁺ par désorption d'ammoniaque.

Le procédé de préparation du catalyseur comprend au moins une étape de mise en forme de la zéolithe traitée avec au moins une matrice et éventuellement un liant. La matrice employée est une matrice minérale poreuse amorphe ou mal cristallisée de type oxyde. Elle est choisie parmi l'alumine, la silice, la silice-alumine, les argiles, notamment les argiles naturelles telles que le kaolin ou la bentonite, la magnésie, l'oxyde de titane, l'oxyde de bore, la zircone, les phosphates d'aluminium, les phosphates de titane, les phosphates de zirconium et le charbon. On peut également choisir une matrice parmi les aluminates. De manière préférée, la matrice est une alumine sous toutes ses formes connues de l'Homme du métier, et de préférence l'alumine gamma. La mise en forme de la zéolithe traitée avec au moins une matrice est généralement telle que le catalyseur se présente sous la forme d'extrudés cylindriques ou polylobés tels que bilobés, trilobés, polylobés de forme droite ou torsadée, mais peut éventuellement être telle que le catalyseur se présente sous la forme de poudres concassées, de tablettes, d'anneaux, de billes, de roues. Les conditions de mise en forme de la zéolithe traitée, le choix de la matrice, éventuellement le broyage préalable de la zéolithe, le procédé de peptisation, l'ajout d'agent porogènes, le temps de malaxage, la pression d'extrusion si le catalyseur est mis sous forme d'extrudés, la vitesse et le temps de séchage sont déterminées pour chaque matrice selon les règles bien connues de l'Homme du métier.
Le procédé de préparation du catalyseur comprend éventuellement une étape de dépôt d'au moins un métal, laquelle est réalisée soit avant la mise en forme, soit lors du mélange de la zéolithe traitée et de la matrice soit encore après la mise en forme. Ledit métal est de préférence un métal choisi dans le groupe VIII de la classification périodique des éléments et plus préférentiellement il s'agit du nickel. La teneur pondérale dudit métal est avantageusement comprise entre 1 et 5% par rapport au poids du catalyseur.

Lorsque l'ajout d'au moins un métal choisi dans le groupe VIII est effectué après la mise en forme, ledit métal peut alors être ajouté, soit avant la calcination, soit, de préférence, après la calcination du mélange matrice-zéolithe traitée. Ledit métal ajouté est généralement déposé, soit pratiquement totalement sur la zéolithe, soit en partie sur la zéolithe et en partie sur la matrice, soit, de préférence, pratiquement totalement sur la matrice, ceci s'effectuant, de la manière qui est connue de l'Homme du métier, par le choix approprié des paramètres utilisés lors dudit dépôt, comme par exemple la nature du précurseur dudit métal. Le dépôt d'au moins un métal du groupe VIII est généralement effectué par la technique d'imprégnation à sec, d'imprégnation par excès, ou de préférence par échange(s) ionique(s).

Par exemple, une des méthodes préférées de préparation du catalyseur consiste à malaxer au moins la zéolithe traitée selon le procédé de traitement dans un gel humide de matrice (obtenu généralement par mélange d'au moins un acide et d'une poudre de matrice), par exemple d'alumine, pendant une durée nécessaire pour l'obtention d'une bonne homogénéité de la pâte ainsi obtenue, soit par exemple pendant une dizaine de minutes, puis à passer ladite pâte à travers une filière pour former des extrudés, par exemple de diamètre compris entre 0,4 et 4 mm bornes incluses, de préférence entre 0,4 et 2,5 mm bornes incluses et de préférence encore entre 0,8 et 2,0 mm bornes incluses. Puis, après séchage pendant quelques heures à environ 120°C en étuve et après calcination, par exemple pendant environ 2 heures à environ 400°C, le métal du groupe VIII est déposé, par exemple par échange ionique en présence d'un agent compétiteur, ledit dépôt étant suivi d'une calcination, par exemple pendant environ 2 heures à environ 400°C.

Le catalyseur préparé et comportant une zéolithe modifiée selon le procédé de traitement, est utilisé dans des procédés de conversion chimique d'hydrocarbures et en particulier dans un procédé d'oligomérisation d'une charge oléfinique contenant des molécules hydrocarbonées ayant de 2 à 12 atomes de carbone par molécule. De manière préférée, la charge utilisée pour la mise en oeuvre dudit procédé d'oligomérisation contient des molécules hydrocarbonées contenant de 3 à 7 atomes de carbone par molécule, et de manière très préférée contenant de 4 à 6 atomes de carbone par molécule. La charge employée dans le procédé d'oligomérisation selon l'invention contient de 20 à 100 % en poids, de préférence de 25 à 80 % en poids et de manière très préférée de 50 à 80 % en poids d'oléfines, les oléfines linéaires représentant de 10 à 100 % en poids, de préférence de 15 à 95 % et de manière très préférée de 50 à 95 % en poids de la totalité des oléfines présentes dans ladite charge.
Des sources possibles pour la charge oléfinique utilisée dans le procédé d'oligomérisation de l'invention sont la coupe légère du craquage en lit fluidisé (fluid catalytic cracking FCC), du vapocraqueur et les effluents d'unités d'éthérification.
De préférence la charge utilisée dans le procédé d'oligomérisation est de type Raffinat II, c'est-à-dire une coupe C4 contenant plus de 50 % en poids d'oléfines linéaires en C4 et moins de 5% en poids d'isobutène, ou une coupe C4 contenant plus de 30% en poids d'oléfines linéaires et moins de 5% en poids d'isobutène, par exemple issue de procédé de production de MTBE ou de TAME ou d'un procédé de type SELECTOPOL (marque déposée), ou une coupe C3/C4 issue d'un procédé de craquage catalytique en lit fluidisé, c'est-à-dire une coupe contenant un mélange propane/propylène dans une proportion en poids d'environ 5/25 et un mélange butane/butène dans une proportion en poids d'environ 25/45.
Ledit procédé d'oligomérisation est préférentiellement mis en oeuvre dans les conditions opératoires suivantes : la pression totale est comprise entre 0,1 et 10 MPa et préférentiellement entre 0,3 et 7 MPa, la température est comprise entre 40 et 600°C et préférentiellement entre 60 et 400°C, la vitesse spatiale horaire (VVH) est comprise entre 0,01 et 100 h⁻¹ et préférentiellement entre 0,4 et 30 h⁻¹.

On précise que, selon l'invention, le procédé d'oligomérisation correspond à une addition limitée à essentiellement 2 à 6 monomères ou molécules de base, lesdits monomères étant des oléfines. L'oligomérisation est considérée comme "essentiellement linéaire" dans la mesure où au moins 75%, de préférence au moins 80% et plus préférentiellement encore au moins 90 % des oligomères obtenus sont linéaires.

Les exemples qui suivent illustrent la présente invention sans en limiter la portée.

### EXEMPLES

### Exemple 1 (invention) : Traitement d'une zéolithe de type structural FER

Pour réaliser la désalumination de la zéolithe H-FER, on procède d'abord à un traitement de 100 g de cette zéolithe (Si/Al = 10,2) pendant 2h à 600°C sous un débit de N₂ contenant 60% poids en H₂O. Puis, la zéolithe résultante est ensuite traitée 2 fois par une solution aqueuse d'acide HNO₃ à 10N (volume solution / volume zéolithe = 5) sous reflux pendant 3 heures, lavée deux fois avec de l'eau distillée, puis séchée. Après rinçage, la zéolithe est ensuite échangée par une solution de NH₄NO₃ sous reflux pendant 2h, puis séchée. La zéolithe échangée est ensuite introduite dans un réacteur à lit fixe où elle est d'abord soumise à une activation sous flux d'azote à 200°C. La température du réacteur est ensuite ramenée à 50°C, puis une pression partielle de 0,15 bar de Si₂H₆ est ajoutée dans le flux d'azote. Après 2h de réaction, la zéolithe est strippée pendant 24h à 120°C pour évacuer le Si₂H₆ non réactif. La décomposition du composé moléculaire Si₂H₆ se fait sous N₂ saturé en H₂0 à 350°C pendant 2 heures, puis un traitement thermique sous N₂ pur est fait à 450°C pendant 2 heures. On obtient ainsi une zéolithe modifiée Z1 (Si/Al = 21,1), sous forme protonée, de type structural FER et comportant une couche de silice amorphe sur ses surfaces externe et interne.

La zéolithe Z1 est ensuite mise en forme par extrusion avec un gel d'alumine de manière à obtenir après séchage à 120 °C et calcination à 450°C sous air sec un catalyseur qui contient 60 % poids de zéolithe Z1 et 40 % poids d'alumine.

### Exemple 2 (invention) : Traitement d'une zéolithe de type structural MFS

Pour réaliser la désalumination de la zéolithe Na-ZSM-57, on procède d'abord à un traitement de 20 g de cette zéolithe (Si/Al = 45,2) pendant 2h à 600°C sous un débit de N₂ contenant 60% poids en H₂O. Puis, la zéolithe résultante est ensuite traitée 2 fois par une solution aqueuse d'acide HNO₃ à 10N (volume solution / volume zéolithe = 5) sous reflux pendant 3 heures, lavée deux fois avec de l'eau distillée, puis séchée. Après rinçage, la zéolithe est ensuite échangée par une solution de NaNO₃ à 80°C pendant 2h, puis séchée. La zéolithe échangée est ensuite introduite dans un réacteur à lit fixe où elle est d'abord soumise à une activation sous flux d'azote à 450°C. La température du réacteur est ensuite ramenée à 50°C, puis une pression partielle de 0,15 bar de Si₂H₆ est ajoutée dans le flux d'azote. Après 2h de réaction, la zéolithe est strippée pendant 24h à 120°C pour évacuer le Si₂H₆ non réactif. La décomposition du composé moléculaire Si₂H₆ se fait sous N₂ saturé en H₂0 à 350°C pendant 2h. Finalement, la zéolithe est échangée par une solution de NH₄NO₃ sous reflux pendant 2h, puis calcinée à 450°C pendant 2h. On obtient ainsi une zéolithe modifiée Z2 (Si/Al = 53,7), sous forme protonée, de type structural MFS et comportant une couche de silice amorphe sur ses surfaces externe et interne.

La zéolithe Z2 est ensuite mise en forme par extrusion avec un gel d'alumine de manière à obtenir après séchage à 120 °C et calcination à 450°C sous air sec un catalyseur qui contient 60 % poids de zéolithe Z2 et 40 % poids d'alumine.

### Exemple 3 (invention) : traitement d'une zéolithe de type structural MOR

Pour réaliser la désalumination de la zéolithe H-MOR, on procède d'abord à un traitement de 100 g de cette zéolithe (Si/Al = 9,1) pendant 2h à 650°C sous un débit de N₂ contenant 60% poids en H₂O. Puis, la zéolithe résultante est ensuite traitée 2 fois par une solution aqueuse d'acide HNO₃ à 8N (volume solution / volume zéolithe = 5) sous reflux pendant 3 heures, lavée deux fois avec de l'eau distillée, puis séchée. Après rinçage, la zéolithe est ensuite échangée par une solution de NH₄NO₃ sous reflux pendant 2h, puis séchée. La zéolithe échangée est ensuite introduite dans un réacteur à lit fixe où elle est d'abord soumise à une activation sous flux d'azote à 200°C. La température du réacteur est ensuite ramenée à 50°C, puis une pression partielle de 0,15 bar de Si₂H₆ est ajoutée dans le flux d'azote. Après 2 h de réaction, la zéolithe est strippée pendant 24 h à 120°C pour évacuer le Si₂H₆ non réactif. La décomposition du composé moléculaire Si₂H₆ se fait sous N₂ saturé en H₂0 à 350°C pendant 2h, puis un traitement thermique sous N₂ pur est fait à 450°C pendant 2 h. On obtient ainsi une zéolithe modifiée Z3 (Si/Al = 25,4), sous forme protonée, de type structural MOR et comportant une couche de silice amorphe sur ses surfaces externe et interne.

La zéolithe Z3 est ensuite mise en forme par extrusion avec un gel d'alumine de manière à obtenir après séchage à 120 °C et calcination à 450°C sous air sec un catalyseur qui contient 60 % poids de zéolithe Z3 et 40 % poids d'alumine.

### Exemple 4 (comparatif) : Traitement d'une zéolithe de type structural FER sans étape de désalumination

On réalise le même traitement que celui donné dans l'exemple 1 en supprimant l'étape de désalumination.

Une quantité de 100 g de zéolithe H-FER (Si/Al = 10,2) a été échangée par une solution de NH₄NO₃ sous reflux pendant 2 h, puis séchée. Après activation de la zéolithe échangée sous flux d'azote dans un réacteur à lit fixe à 200°C, la température du réacteur est ramenée à 50°C, puis une pression partielle de 0,15 bar de Si₂H₆ est ajoutée dans le flux d'azote. Après 2h de réaction, la zéolithe est strippée pendant 24h à 120°C pour évacuer le Si₂H₆ non réactif. La décomposition du composé moléculaire Si₂H₆ se fait sous N₂ saturé en H₂0 à 350°C pendant 2h, puis un traitement thermique sous N₂ pur est fait à 450°C pendant 2h. On obtient ainsi une zéolithe Z1d de type structural FER, non désaluminée, et comportant une couche de silice amorphe sur ses surfaces externe et interne.

La zéolithe Z1d est ensuite mise en forme par extrusion avec un gel d'alumine de manière à obtenir après séchage à 120 °C et calcination à 450°C sous air sec un catalyseur qui contient 60 % poids de zéolithe Z1d et 40 % poids d'alumine.

### Exemple 5 (comparatif) : Traitement d'une zéolithe de type structural MFS sans étape de désalumination

On réalise le même traitement que celui donné dans l'exemple 2 en supprimant l'étape de désalumination.
Une quantité de 20 g de zéolithe Na-ZSM-57 (Si/Al = 45,2), déjà sous forme non acide, a été activée sous flux d'azote dans un réacteur à lit fixe à 450°C, puis la température du réacteur est ramenée à 50°C et une pression partielle de 0,15 bar de Si₂H₆ est ajoutée dans le flux d'azote. Après 2 heures de réaction, la zéolithe est strippée pendant 24 heures à 120°C pour évacuer le Si₂H₆ non réactif. La décomposition du composé moléculaire Si₂H₆ se fait sous N₂ saturé en H₂0 à 350°C pendant 2 heures. Finalement, la zéolithe est échangée par une solution de NH₄NO₃ sous reflux pendant 2h, puis calcinée à 450°C pendant 2h. On obtient ainsi une zéolithe Z2d de type structural MFS, non désaluminée, et comportant une couche de silice amorphe sur ses surfaces externe et interne.

La zéolithe Z2d est ensuite mise en forme par extrusion avec un gel d'alumine de manière à obtenir après séchage à 120 °C et calcination à 450°C sous air sec un catalyseur qui contient 60 % poids de zéolithe Z2d et 40 % poids d'alumine.

### Exemple 6 (comparatif) : Traitement d'une zéolithe de type structural MOR sans étape de désalumination

On réalise le même traitement que celui donné dans l'exemple 3 en supprimant l'étape de désalumination.
Une quantité de 100 g de zéolithe H-MOR (Si/Al = 9,1) a été échangée par une solution de NH₄NO₃ sous reflux pendant 2h, puis séchée. Après activation de la zéolithe échangée sous flux d'azote dans un réacteur à lit fixe à 200°C, la température du réacteur est ramenée à 50°C, puis une pression partielle de 0,15 bar de Si₂H₆ est ajoutée dans le flux d'azote. Après 2h de réaction, la zéolithe est strippée pendant 24 heures à 120°C pour évacuer le Si₂H₆ non réactif. La décomposition du composé moléculaire Si₂H₆ se fait sous N₂ saturé en H₂0 à 350°C pendant 2h, puis un traitement thermique sous N₂ pur est fait à 450°C pendant 2 heures. On obtient ainsi une zéolithe Z3d de type structural MOR, non désaluminée, et comportant une couche de silice amorphe sur ses surfaces externe et interne.

La zéolithe Z3d est ensuite mise en forme par extrusion avec un gel d'alumine de manière à obtenir après séchage à 120 °C et calcination à 450°C sous air sec un catalyseur qui contient 60 % poids de zéolithe Z3d et 40 % poids d'alumine.

### Exemple 7 (comparatif) : Traitement d'une zéolithe de type structural FER sans étape d'échange cationique

On réalise le même traitement que celui donné dans l'exemple 1 en supprimant l'étape d'échange cationique préalable à l'étape de traitement en présence du composé moléculaire Si₂H₆.
Une quantité de 100 g de zéolithe H-FER (Si/Al = 10,2) a été traitée pendant 2 heures à 600°C sous un débit de N₂ contenant 60% poids en H₂O. Puis, la zéolithe résultante est ensuite traitée 2 fois par une solution aqueuse d'acide HNO₃ à 10N (volume solution / volume zéolithe = 5) sous reflux pendant 3 heures, lavée deux fois avec de l'eau distillée, puis séchée. Après activation de la zéolithe désaluminée sous flux d'azote dans un réacteur à lit fixe à 200°C, la température du réacteur est ramenée à 50°C, puis une pression partielle de 0,15 bar de Si₂H₆ est ajoutée dans le flux d'azote. Après 2 heures de réaction, la zéolithe est strippée pendant 24 heures à 120°C pour évacuer le Si₂H₆ non réactif. La décomposition du composé moléculaire Si₂H₆ se fait sous N₂ saturé en H₂0 à 350°C pendant 2 heures, puis un traitement thermique sous N₂ pur est fait à 450°C pendant 2 heures. On obtient ainsi une zéolithe Z1e de type structural FER comportant une couche de silice amorphe sur ses surfaces externe et interne.

La zéolithe Z1e est ensuite mise en forme par extrusion avec un gel d'alumine de manière à obtenir après séchage à 120 °C et calcination à 450°C sous air sec un catalyseur qui contient 60 % poids de zéolithe Z1e et 40 % poids d'alumine.

### Exemple 8 (comparatif) : Traitement d'une zéolithe de type structural MFS sans étape d'échange cationique

On réalise le même traitement donné dans l'exemple 2 en supprimant l'étape d'échange cationique préalable à l'étape de traitement en présence du composé moléculaire Si₂H₆.
Une quantité de 20 g de zéolithe Na-ZSM-57 (Si/Al = 45,2) a été traitée pendant 2h à 600°C sous un débit de N₂ contenant 60% poids en H₂O. Puis, la zéolithe résultante est ensuite traitée 2 fois par une solution aqueuse d'acide HNO₃ à 10N (volume solution / volume zéolithe = 5) sous reflux pendant 3 heures, lavée deux fois avec de l'eau distillée, puis séchée. Après activation de la zéolithe désaluminée sous flux d'azote dans un réacteur à lit fixe à 450°C, la température du réacteur est ramenée à 50°C, puis une pression partielle de 0,15 bar de Si₂H₆ est ajoutée dans le flux d'azote. Après 2h de réaction, la zéolithe est strippée pendant 24 heures à 120°C pour évacuer le Si₂H₆ non réactif. La décomposition du composé moléculaire Si₂H₆ se fait sous N₂ saturé en H₂0 à 350°C pendant 2 heures. Finalement, la zéolithe est échangée par une solution de NH₄NO₃ sous reflux pendant 2 heures, puis calcinée à 450°C pendant 2 heures. On obtient ainsi une zéolithe Z2e de type structural MFS comportant une couche de silice amorphe sur ses surfaces externe et interne.

La zéolithe Z2e est ensuite mise en forme par extrusion avec un gel d'alumine de manière à obtenir après séchage à 120 °C et calcination à 450°C sous air sec un catalyseur qui contient 60 % poids de zéolithe Z2e et 40 % poids d'alumine.

### Exemple 9 (comparatif) : Traitement d'une zéolithe de type structural MOR sans étape d'échange cationique

On réalise le même traitement donné dans l'exemple 3 en supprimant l'étape d'échange cationique préalable à l'étape de traitement en présence du composé moléculaire Si₂H₆.
Une quantité de 100 g de zéolithe H-MOR (Si/Al = 9,1) a été traitée pendant 2 heures à 650°C sous un débit de N₂ contenant 60% poids en H₂O. Puis, la zéolithe résultante est ensuite traitée 2 fois par une solution aqueuse d'acide HNO₃ à 8N (volume solution / volume zéolithe = 5) sous reflux pendant 3 heures, lavée deux fois avec de l'eau distillée, puis séchée. Après activation de la zéolithe dasaluminée sous flux d'azote dans un réacteur à lit fixe à 200°C, la température du réacteur est ramenée à 50°C, puis une pression partielle de 0,15 bar de Si₂H₆ est ajoutée dans le flux d'azote. Après 2 heures de réaction, la zéolithe est strippée pendant 24 heures à 120°C pour évacuer le Si₂H₆ non réactif. La décomposition du composé moléculaire Si₂H₆ se fait sous N₂ saturé en H₂0 à 350°C pendant 2h, puis un traitement thermique sous N₂ pur est fait à 450°C pendant 2h. On obtient ainsi une zéolithe Z3e de type structural MOR comportant une couche de silice amorphe sur ses surfaces externe et interne.

La zéolithe Z3e est ensuite mise en forme par extrusion avec un gel d'alumine de manière à obtenir après séchage à 120 °C et calcination à 450°C sous air sec un catalyseur qui contient 60 % poids de zéolithe Z3e et 40 % poids d'alumine.

### Exemple 10 : Evaluation des propriétés catalytiques des différents catalyseurs en oligomérisation des oléfines légères.

Les performances des catalyseurs préparés selon les exemples 1 à 9 ci-dessus ont été évalués dans l'oligomérisation d'une coupe oléfinique légère contenant 54% de butènes et 4 % d'isobutène dans un mélange de paraffines.

Les conditions opératoires de tests sont les suivantes:
Température : 230°C
Pression : 6 MPa
VVH (h⁻¹) [volume de catalyseur / débit volumique de charge] : 1 h⁻¹

Les catalyseurs sont préalablement activés in situ sous N₂ à 450°C pendant 2h.

Les performances des catalyseurs à base de la zéolithe de type structural FER sont données dans le Tableau 1.

**Tableau 1 : Performances des catalyseurs à base de la zéolithe FER.**

| | Catalyseur à base de Z1 | Catalyseur à base de Z1d | Catalyseur à base de Z1e |
|---|---|---|---|
| Conversion C4 oléfinique (%) | 69 | 27 | 3 |
| Rendement coupe essence (%) | 31 | 11 | < 2% |
| Rendement coupe diesel (%) | 29 | 4 | - |
| Indice de cétane | 47 | 35 | - |

Les performances des catalyseurs à base de la zéolithe ZSM-57 de type structural MFS sont données dans le Tableau 2.

**Tableau 2 : Performances des catalyseurs à base de la zéolithe ZSM-57.**

| | Catalyseur à base de Z2 | Catalyseur à base de Z2d | Catalyseur à base de Z2e |
|---|---|---|---|
| Conversion C4 oléfinique (%) | 72 | 32 | 67 |
| Rendement coupe essence (%) | 33 | 13 | 31 |
| Rendement coupe diesel (%) | 31 | 2 | 26 |
| Indice de cétane | 48 | - | 47 |

Les performances des catalyseurs à base de la zéolithe de type structural MOR sont données dans le Tableau 3.

**Tableau 3 : Performances des catalyseurs à base de la zéolithe MOR.**

| | Catalyseur à base de Z3 | Catalyseur à base de Z3d | Catalyseur à base de Z3e |
|---|---|---|---|
| Conversion C4 oléfinique (%) | 99 | 31 | < 2 |
| Rendement coupe essence (%) | 38 | 10 | - |
| Rendement coupe diesel (%) | 57 | 6 | - |
| Indice de cétane | 41 | 33 | - |

Les performances catalytiques présentées dans les tableaux 1, 2 et 3 démontrent que le catalyseur comprenant une zéolithe modifiée et préparée selon le procédé de traitement permet d'augmenter notablement l'activité du catalyseur pour la conversion des C4 oléfinique, augmentant ainsi les rendements en coupes essence et diesel. La qualité de ce gazole, mesuré par son indice de cétane (IC) est aussi amélioré par rapport à celui présenté par une coupe diesel obtenue au moyen d'un catalyseur comprenant une zéolithe qui n'a pas été traitée selon le procédé de traitement de l'invention. Il est manifeste que les étapes de désalumination et d'échange cationique sont essentielles dans le procédé selon l'invention.

## Revendications

1. Procédé d'oligomérisation d'une charge oléfinique contenant des molécules hydrocarbonées ayant de 2 à 12 atomes de carbone par molécule réalisé en présence d'un catalyseur contenant une zéolithe traitée laquelle est préparée selon un procédé de traitement comportant au moins :
a) une étape de désalumination d'une zéolithe choisie parmi les zéolithes de type structural MEL, MFI, ITH, NES, EUO, ERI, FER, CHA, MFS, MWW, MTT, TON et MOR présentant une taille de pores inférieure ou égale à 7 À, la zéolithe à l'issue de ladite étape a) se présentant sous forme cationique et est débarrassée de structurant organique,
b) une étape d'échange cationique par au moins un cation autre que H⁺ choisi parmi les cations Na⁺, Li⁺, K⁺, Rb⁺, Cs⁺, Ba²⁺, Ca²⁺ et NH₄⁺,
c) une étape de traitement de ladite zéolithe obtenue à l'étape b) en présence d'au moins un composé moléculaire contenant au moins un atome de silicium choisi parmi les composés de formule Si-R4 et Si2-R6 où R peut être soit de l'hydrogène, soit un groupe alkyle, aryle ou acyle, soit un groupe alkoxy (O-R'), soit un groupe hydroxyl (-OH) soit encore un halogène, de type silane, disilane, alkylsilane, alkoxysilane ou siloxane,
d) au moins une étape de traitement thermique,
et dans lequel ledit catalyseur est préparé par un procédé comprenant au moins une étape de mise en forme de ladite zéolithe traitée avec au moins une matrice.

2. Procédé d'oligomérisation selon la revendication 1 tel que ladite zéolithe présente initialement un rapport atomique Si/Al compris entre 2 et 200.

3. Procédé d'oligomérisation selon l'une des revendications 1 ou 2 tel que ladite zéolithe se présente, préalablement à la mise en oeuvre de ladite étape a) dudit procédé de traitement, sous forme calcinée et contient au moins un cation.

4. Procédé d'oligomérisation selon l'une des revendications 1 à 3 tel que le cation employé pour réaliser ladite étape b) est un cation ammonium NH₄⁺.

5. Procédé d'oligomérisation selon l'une des revendications 1 à 4 tel que ladite étape c) dudit procédé de traitement est réalisée en procédant au dépôt dudit composé moléculaire contenant au moins un atome de silicium en phase gazeuse.

6. Procédé d'oligomérisation selon la revendication 5 tel que ladite étape c) est réalisée dans un réacteur à lit fixe.

7. Procédé d'oligomérisation selon l'une des revendications 1 à 6 tel que ladite étape d) dudit procédé de traitement est suivie d'une étape e) d'échange dudit cation par un cation ammonium, elle-même suivie d'une étape f) consistant en un traitement thermique.

8. Procédé d'oligomérisation selon l'une des revendications 1 à 7 tel que ledit catalyseur est préparé par un procédé comprenant en outre une étape de dépôt d'au moins un métal.

9. Procédé d'oligomérisation selon l'une des revendications 1 à 8 tel que ladite charge contient de 25 à 80 % poids d'oléfines, les oléfines linéaires représentant 15 à 95 % en poids de la totalité des oléfines présentes dans ladite charge.

10. Procédé d'oligomérisation selon l'une des revendications 1 à 9 tel qu'il est mis en oeuvre à une température comprise entre 40 et 600°C, avec une pression totale comprise entre 0,1 et 10 MPa et une vitesse spatiale horaire (VVH) comprise entre 0,01 et 100 h⁻¹.

## Patentansprüche

1. Verfahren zur Oligomerisation einer Olefincharge, die Kohlenwasserstoffmoleküle mit 2 bis 12 Kohlenstoffatomen pro Molekül enthält, welches in Anwesenheit eines Katalysators durchgeführt wird, der einen behandelten Zeolith enthält, welcher gemäß einem Behandlungsverfahren hergestellt wird, umfassend mindestens:
a) einen Schritt des Desalumination eines Zeoliths, ausgewählt aus Zeolithen vom Strukturtyp MFL, MFI, ITH, NES, EUO, ERI, FER, CHA, MFS, MWW, MTT, TON und MOR, mit einer Porengröße kleiner oder gleich 7 Ä, wobei sich der Zeolith am Ende des Schritts a) in kationischer Form präsentiert und frei ist von einem organischen Strukturierungsmittel,
b) einen Schritt eines Kationenaustauschs durch mindestens ein anderes Kation als H⁺, ausgewählt aus den Kationen Na⁺, Li⁺, K⁺, Rb⁺, Ca⁺, Ba²⁺, Ca²⁺ und NH₄⁺,
c) einen Schritt der Behandlung des in Schritt b) erhaltenen Zeoliths in Anwesenheit mindestens einer molekularen Verbindung, die mindestens ein Siliciumatom enthält, ausgewählt aus den Verbindungen mit der Formel Si-R4 und Si2-R6, wobei R entweder Wasserstoff, oder eine Alkyl-, Aryl- oder Acylgruppe, oder eine Alkoxygruppe (O-R'), oder eine Hydroxylgruppe (-OH), oder auch ein Halogen, vom Typ Silan, Disilan, Alkylsilan, Alkoxysilan oder Siloxan, sein kann,
d) mindestens einen thermischen Behandlungsschritt,
und wobei der Katalysator durch ein Verfahren hergestellt wird, umfassend mindestens einen Schritt der Formung des behandelten Zeoliths mit mindestens einer Matrix.

2. Verfahren zur Oligomerisation nach Anspruch 1, so dass der Zeolith anfänglich ein Atomverhältnis Si/Al zwischen 2 und 200 aufweist.

3. Verfahren zur Oligomerisation nach den Ansprüchen 1 oder 2, so dass sich der Zeolith, vor der Durchführung des Schritts a) des Behandlungsverfahrens, in calcinierter Form präsentiert und mindestens ein Kation enthält.

4. Verfahren zur Oligomerisation nach einem der Ansprüche 1 bis 3, so dass das Kation, das zur Durchführung des Schritts b) verwendet wird, ein Ammoniumkation NH₄⁺ ist.

5. Verfahren zur Oligomerisation nach einem der Ansprüche 1 bis 4, so dass der Schritt c) des Behandlungsverfahrens durchgeführt wird, indem die Abscheidung der molekularen Verbindung, die mindestens ein Siliciumatom enthält, in der Gasphase vorgenommen wird.

6. Verfahren zur Oligomerisation nach Anspruch 5, so dass der Schritt c) in einem Festbett-Reaktor durchgeführt wird.

7. Verfahren zur Oligomerisation nach einem der Ansprüche 1 bis 6, so dass der Schritt d) des Behandlungsverfahrens von einem Schritt e) eines Austauschs des Kations durch ein Ammoniumkation gefolgt wird, der selbst von einem Schritt f) gefolgt wird, bestehend aus einer thermischen Behandlung.

8. Verfahren zur Oligomerisation nach einem der Ansprüche 1 bis 7, so dass der Katalysator durch ein Verfahren hergestellt wird, umfassend außerdem einen Schritt der Abscheidung mindestens eines Metalls.

9. Verfahren zur Oligomerisation nach einem der Ansprüche 1 bis 8, so dass die Charge 25 bis 80 Gew.-% Olefine enthält, wobei lineare Olefine 15 bis 95 Gew.-% der Gesamtheit der in der Charge vorhandenen Olefine ausmachen.

10. Verfahren zur Oligomerisation nach einem der Ansprüche 1 bis 9, so dass dieses bei einer Temperatur zwischen 40 und 600°C und mit einem Gesamtdruck zwischen 0,1 und 10 MPa und einer Raumgeschwindigkeit pro Stunde (VVH) zwischen 0,01 und 100 h⁻¹ durchgeführt wird.

## Claims

1. A process for the oligomerisation of a olefinic charge containing hydrocarbon molecules having from 2 to 12 carbon atoms per molecule operating in presence of a catalyst containing a treated zeolite which has been prepared according to a process of treatment comprising at least:
a) a step for dealumination of a zeolite selected from the zeolites of structural type MEL, MFI, ITH, NES, EUO, ERI, FER, CHA, MFS, MWW, MTT, TON and MOR having a pore size of less than or equal to 7Å, said zeolithe, at the outcome of the dealumination step a) is in cationic form, and is freed of the organic structuring agent,
b) a cationic exchange step using at least one cation other than H⁺, selected from the cations Na+, Li+, K+, Rb+, Cs+, Ba2+ , Ca2+ and NH4+,
c) a step for treatment of said zeolite obtained in step b) in the presence of at least one molecular compound containing at least one silicon atom, selected from the compounds of the formulae Si-R₄ and Si2-R6 wherein R can be either hydrogen or an alkyl, aryl or acyl group, or an alkoxy group (O-R') or a hydroxyl group (-OH) or again a halogen, a compound of silane, disilane, alkylsilane, alkoxysilane or siloxane type and
d) at least one heat treatment step.

2. A process for the oligomerisation according to claim 1 such that said zeolite initially has an Si/Al atomic ratio of between 2 and 200.

3. A process for the oligomerisation according to one of claims 1 or 2 such that said zeolite, prior to implementation of said step a), is in calcined form and contains at least one cation.

4. A process for the oligomerisation according to one of claims 1 to 3 such that the cation employed for implementing said step b) is an ammonium cation NH₄⁺.

5. A process for the oligomerisation according to one of claims 1 to 4 such that step c) is implemented by effecting the deposit of said molecular compound comprising at least one silicon atom in a gaseous phase.

6. A process for the oligomerisation according to claim 5 such that said step c) is implemented in a fixed bed reactor.

7. A process for the oligomerisation according to claim 1 to 6 such that step d) is followed by a step e) for the exchange of said cation by an ammonium cation, itself followed by a step f) consisting of a heat treatment.

8. A process for the oligomerisation according to one of claims 1 to 7 such that said catalyst is prepared by a process further comprising a step of depositing at least one metal..

9. A process for the oligomerisation according to claim 1 to 8 such that said said charge contains from 25 to 80 % by weight of olefins, the linear olefins representing 15 to 95 % by weight of all of the olefins present in said charge.

10. A process for the oligomerisation according to one of claims 1 to 9 such that said oligomerisation process is carried out at a temperature of between 40 and 600oC, with a total pressure of between 0.1 and 10 MPa and a spatial hourly velocity (VVH) of between 0.01 and 100 h-1.
